(19) Europäisches Patentamt / European Patent Office / Office européen des brevets

(11) **EP 4 069 666 B1**

(12) **EUROPEAN PATENT SPECIFICATION**

(45) Date of publication and mention
of the grant of the patent:
**21.08.2024 Bulletin 2024/34**

(21) Application number: **20816495.4**

(22) Date of filing: **04.12.2020**

(51) International Patent Classification (IPC):
**C07C 37/54** [(2006.01)]

(52) Cooperative Patent Classification (CPC):
(C-Sets available)
**C07C 37/54** (Cont.)

(86) International application number:
**PCT/EP2020/084673**

(87) International publication number:
**WO 2021/110933 (10.06.2021 Gazette 2021/23)**

(54) **METHOD FOR PREPARATION OF LIGNIN OLIGOMERS**

VERFAHREN ZUR HERSTELLUNG VON LIGNINOLIGOMEREN

PROCÉDÉ DE PRÉPARATION D'OLIGOMÈRES DE LIGNINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2019 EP 19214133**

(43) Date of publication of application:
**12.10.2022 Bulletin 2022/41**

(73) Proprietor: **BASF SE**
**67056 Ludwigshafen am Rhein (DE)**

(72) Inventors:
• **KINDLER, Alois**
**67056 Ludwigshafen am Rhein (DE)**
• **LINDNER, Jean-Pierre Berkan**
**67056 Ludwigshafen am Rhein (DE)**
• **WITTICH, Knut**
**76297 Stutensee (DE)**
• **ROMANENKO, Yuliia**
**69123 Heidelberg (DE)**
• **SCHUNK, Stephan A.**
**69123 Heidelberg (DE)**

(74) Representative: **BASF IP Association**
**BASF SE**
**GBI-C006**
**67056 Ludwigshafen (DE)**

(56) References cited:
**EP-A1- 2 975 015    WO-A2-2014/201325**

• **KONNERTH HANNELORE ET AL: "Base promoted hydrogenolysis of lignin model compounds and organosolv lignin over metal catalysts in water", CHEMICAL ENGINEERING SCIENCE, OXFORD, GB, vol. 123, 4 November 2014 (2014-11-04), pages 155 - 163, XP029131462, ISSN: 0009-2509, DOI: 10.1016/J.CES.2014.10.045**
• **CHENG FENG ET AL: "Producing jet fuel from biomass lignin: Potential pathways to alkyl-benzenes and cycloalkanes", RENEWABLE AND SUSTAINABLE ENERGY REVIEWS, ELSEVIERS SCIENCE, NEW YORK, NY, US, vol. 72, 21 January 2017 (2017-01-21), pages 673 - 722, XP029963412, ISSN: 1364-0321, DOI: 10.1016/J.RSER.2017.01.030**

(52) Cooperative Patent Classification (CPC): (Cont.)

C-Sets
**C07C 37/54, C07C 39/12**

**Description**

The present invention relates a process for depolymerization of lignin comprising thermal treatment of an aqueous mixture having a pH of at least 9 comprising lignin, catalyst and primary alcohol in a non-oxidizing atmosphere at a temperature of at least 280°C.

Chemical industry needs aromatics as building blocks for a variety of products, e.g. polymers. Renewables based aromatics are potentially available by lignin depolymerization.

In the concept of sustainable chemistry, the valorization of lignin is of significant importance as it is the second largest renewable resource after cellulose. Additional interest arises as lignin is in principle readily available as by-product of the pulp industry and paper industry. Currently, lignin is simply burned and its energy is recovered and used to run the paper production process. Since paper industries are becoming more efficient and requires less energy consumption the news strategies of lignin valorization get considerable attention.

However, lignin valorization has some obstacles and the known processes require several steps for lignin depolymerization and isolation. Moreover, harsh reaction conditions are required for lignin depolymerization, which make utilization of catalyzed reaction steps desirable. However, usually high sulfur content of lignin from the pulp & paper industry (around 2-3%) is challenging for catalyst lifetimes. Additionally, lignin depolymerization often results in formation of highly condensed lignin-species in the form of insoluble solid char-like residues. Besides the fact that such char-like solid residue decreases yield of desired products reactor clogging is a big problem for industrial applications which are to be avoided. Further, known methods do not result in a high yield of monoaromatic or oligomeric aromatic structures with low molecular mass of < 700 g/mol and/or narrow molecular size distribution.

WO2017/048163 describes a complex multi-step process for bio-oil production from black liquor in the presence of an acidifying agent under $H_2$ or $H_2/CO$ pressure of 5-150 bar, at 180 to 240 °C for 10-120 minutes in the presence of a solid catalyst. The depolymerized lignin fraction is recovered by acidification till pH 4-5 followed by extraction.

WO2017/078582 describes a catalytic process for conversion of lignin oil (of molecular weight average of 500 g/mol to 800 g/mol) to a hydrocarbon product by treatment of lignin oil under $H_2$ pressure of 30-160 bar, at 290 to 400°C and subsequent recovery of the hydrogenated products by gas/liquid phase separation.

WO2014/168473 describes a process of lignin depolymerization in alkaline media in the presence of noble metal supported catalysts at 200-300°C without addition of alcohol to obtain phenolic compounds. The process is not high in yield of desired low molecular weight lignin species.

WO2012/005677 describes a process of obtaining lignin with lower molecular weight, lower viscosity and higher purity by lowering pH of black liquor and treating black liquor at 150-200 °C for 1-60 min. However, molecular weight is just reduced from 4810 g/mol to 4050 g/mol.

EP2975015 describes a process of lignin depolymerization at temperatures of 230 to 350 °C in the presence of a subgroup VI element catalyst with water and/or ethanol as solvent in the absence of a base. By this reaction a liquid product is obtained.

WO2014/201325 describes a process for lignin depolymerization at temperatures below 220°C in the presence of a metaloxide catalyst with methanol as solvent in the absence of a base. By this reaction a depolymerized lignin oil is obtained which is further purified by extraction.

Konnerth et al. "Base promoted hydrogenolysis of lignin model compounds and organosolv lignin over metal catalysts in water", Chemical Engineering Science 123 (2015), 155 - 163, describes lignin depolymerization at 130 and 160 °C in the presence of Ru and Ni catalysts with water as solvent at pH 12 to 13. By this reaction a depolymerized lignin material is obtained which is further purified by extraction.

Cheng et al. "Producing jet fuel from biomass lignin: Potential pathways to alkylbenzenes and cycloalkanes", Renewable and Sustainable Energy Reviews 72(2017) 673 - 722, is a review describing lignin hydrogenolysis at a temperature of 200 to 600°C resulting in a liquid oil with an molecular weight from 150 to 300 Da in water/ethanol 1:1 and mentions drastically reduced yields when using pure ethanol.

It is a primary object of the invention to provide a simple process for depolymerization of lignin to oligomers having a molecular weight below 1000 g/mol in high yield and a narrow molecular size distribution as well as to avoid or reduce formation of char-type lignin residues.

This object is achieved by a process for depolymerization of lignin by thermal conversion of an aqueous mixture having a pH of at least 9 comprising lignin, catalyst and primary alcohol in a non-oxidizing atmosphere at a temperature of at least 280°C.

The process for depolymerization of lignin according to the present invention may comprise the steps of:

a) Providing an aqueous mixture having a pH of at least 9 comprising:

lignin, catalyst and primary alcohol in a non-oxidizing atmosphere,

b) Thermal conversion of the lignin in the aqueous mixture at a temperature of at least 280°C, and

c) Obtaining lignin oligomer as a precipitate.

Advantageously, the lignin oligomer is obtained as a precipitate which can be easily separated from the reaction solution.

The thermal conversion in step b) may be performed for a time until precipitation of lignin oligomer. Preferably, the thermal conversion in step b) is performed for 3 to 7 h, more preferably 4 to 5 h, after precipitation of lignin oligomer starts. The obtained lignin oligomer precipitate may be filtered from the aqueous reaction solution. Optionally, precipitation may be performed by cooling the aqueous mixture after thermal conversion. Cooling temperature may be between 50 °C and 15°C, more preferably between 40 and 20 °C. Preferably, the temperature is cooled to below 20°C, more preferably below 15 °C but preferably the temperature is not cooled below 0°C.

The obtained lignin oligomer is re-dissolvable in organic solvents, like e.g. alcohol, acetone, THF and/or DMSO. Advantageously, during the process according to the present invention formation of char-type lignin residues is reduced or even avoided. Char-type lignin residues means insoluble, high molecular weight (> 5000 g/mol) highly condensed lignin-based material.

The aqueous mixture in the process according to the present invention may comprise

5 to 25 wt.-% lignin,

0.01 to 5 wt.-% catalyst, preferably 0.01 to 1.5 wt.-% catalyst, and

5 to 45 wt.-% primary alcohol, preferably 10 to 20 wt.-% primary alcohol,

based on the total weight of the aqueous mixture.

The addition of primary alcohol is required to reduce or avoid formation of char-type lignin residues during the thermal conversion. However, primary alcohol amount should not be too high to allow efficient product recovery by precipitation of the desired lignin oligomer. Surprisingly, in the range of 5 to 45 wt.-%, preferably 10 to 20 wt.-%, primary alcohol based on the total weight of the aqueous mixture, precipitation of desired lignin occurs without additional formation of char-type lignin residues.

The lignin oligomer obtained by the process according to the present invention precipitates as a soft, rubbery type material that can be re-dissolved in alcohol (e.g. ethanol), acetone, THF, and/or DMSO after the aqueous reaction mixture has been removed. The aqueous reaction solution and the precipitated lignin oligomer can be separated by e.g. decanting, centrifugation and/or filtration or other types of solids-removal technologies known in the art.

The catalyst used in the process according to the present invention may be a heterogenous catalyst, preferably for deoxydehydration. The catalyst used in the process according to the present invention may be selected from the group consisting of Ru, Cu, Co, Ni, Pt, sulfides of said metals, oxides of said metals and mixtures thereof. A carbon and/or $Al_2O_3$ and/or $SiO_2$ support may be used for the catalyst. Preferably Ru on a C-carrier, Ni-nanoparticles or Cu on an $Al_2O_3/SiO_2$ support are used according to the present invention. Optionally, the catalyst may be recycled and reused in the process according to the present invention. The obtained precipitated lignin oligomer may be dissolved in organic solvents, like alcohol (e.g. ethanol), acetone, THF, and/or DMSO. The catalyst included in the precipitate remains undissolved and may be separated by e.g. decanting, centrifugation and/or filtration or other types of solids-removal technologies known in the art. Surprisingly, the catalyst after use in the process according to the present invention has a prolonged life-time compared to the same catalyst used in processes for lignin depolymerization known in the art.

The primary alcohol used in the process according to the present invention preferably is a primary C1 to C4-alcohol and may be selected from the group consisting of MeOH, EtOH, n-Propanol, n-Butanol and mixtures thereof. Preferably, EtOH is used as alcohol in the process according to the present invention.

However, the process according to the present invention does not require aromatic solvents. Preferably, no aromatic solvents are used in the process according to the present invention.

Furthermore, preferably the process according to the present invention does not require an extraction step and/or an adsorption step and/or an ion exchange step.

Preferably, the process according to the present invention does not comprise an acidification step. However, a base may be added to adjust pH of the aqueous solution to a pH of at least 9. The base which may be used for the process according to the present invention may be selected from the group consisting of NaOH, KOH, $Ca(OH)_2$, $Mg(OH)_2$ and mixtures thereof. Preferably, NaOH is used as a base according to the present invention.

The non-oxidizing gas used in the process according to the present invention may be selected from the group consisting $N_2$, argon and/or $H_2$. Preferably, the non-oxidizing gas is $N_2$. Preferably, the process according to the present invention is performed in the absence of hydrogen gas. $N_2$-gas provides higher yields than $H_2$-gas.

The process according to the present invention may be conducted at 280°C to 400°C, preferably 280°C to 320°C. Within this temperature range the reaction can be better controlled and formation of char-like residue is reduced or avoided. Advantageously, depolymerization of lignin and deoxydehydration of lignin are both performed during the thermal conversion, preferably in one step. Furthermore, the depolymerized lignin precipitates under said reaction conditions.

Preferably the reaction is performed at a reaction pressure of 80 bar to 150 bar, more preferably 90 bar to 120 bar in the non-oxidizing atmosphere.

In one embodiment of the process according to the present invention is conducted at a reaction pressure of 80 bar to 150 bar, preferably 90 to 120 bar and 280 to 320°C, in the presence of a non-oxidizing atmosphere preferably selected form the group consisting of $N_2$, argon, $H_2$ and mixtures thereof.

In a preferred embodiment the process for depolymerization of lignin according to the present invention comprises the steps of:

a) Providing an aqueous mixture having a pH of at least 9 comprising:
lignin, catalyst and primary alcohol in a non-oxidizing atmosphere,
b) Thermal conversion of the lignin in the aqueous mixture, and
c) Obtaining lignin oligomer,

wherein the aqueous mixture in the process according to the present invention may comprise

5 to 25 wt.-% lignin, preferably 15 to 25 wt.-lignin,
0.01 to 5 wt.-% catalyst, preferably 0.01 to 1.5 wt.-% catalyst
1 to 5 wt.-% base NaOH and/or KOH and
5 to 45 wt.-% primary alcohol, preferably 10 to 20 wt.-% primary alcohol,
based on the total weight of the aqueous mixture,
wherein the thermal conversion is performed at a temperature of 280 to 320°C, at a reaction pressure of 80 bar to 150 bar, preferably 90 to 120 bar, in a H2- or N2-non-oxidizing atmosphere and/or
preferably for 5 to 7 h and/or
preferably using Ru/C catalyst, and/or
preferably using EtOH as primary alcohol, and/or
wherein preferably the obtained lignin oligomer has a molecular weight of 250 to 750 g/mol, preferably 300 to 650 g/mol, more preferably 300 to 500 g/mol and/or a size distribution of below 3.

Preferably the precipitated lignin oligomer is separated from the aqueous reaction solution by decanting, centrifugation and/or filtration, then the lignin oligomer is dissolved in a primary alcohol, e.g. ethanol, and subsequently the catalyst is separated by decanting, centrifugation and/or filtration, wherein the separated aqueous reaction solution comprising unreacted or partially depolymerized and deoxydehydrated lignin, base, and primary alcohol is reintroduced into step a). Preferably, additional lignin equal to the mass of recovered product in the previous reaction cycle is added to the aqueous mixture in the new cycle.

Further, the separated catalyst may be reintroduced reintroduced into step a).

Lignin is a high-molecular weight, aromatic compound found in plants comprising hydroxylated and methoxylated phenylpropene units like 4-hydroxycinnamic alcohol (p-cumaryl alcohol), co-niferyl alcohol and/or sinapyl alcohol, (so-called monolignols) units.

The lignin according to the present invention may be obtained e.g. by the sulfate process (Kraft lignin), soda process and/or organosolv-process (Organosolv lignin). Processes to obtain lignin are e.g. described in US4507172, CA2256923, EP3156409, WO2013/070130, DE3901662, WO2012/027767 and/or WO2006/038863.

Lignin may be also precipitated as lignin solid out of a Kraft pulp mill "black liquor" stream by acidification and filtration (e.g. by the Lignoboost process described in US20170355723 or equivalent approaches). Preferably, this type of lignin is used according to the present invention and referred to as Kraft lignin.

Black liquor is the aqueous basic solution of the Kraft-pulping process after separation of the cellulosic pulp. It comprises besides dissolved lignin inorganic cooking salts and degraded sugar components from the original biomass, like e.g. acetic acid, diverse sugar-acids, etc. (Bajpai, Pratima. (2018). Biermann's Handbook of Pulp and Paper - Raw Material and Pulp Making, Volume 1 and 2 (3rd Edition) - 12.8.5 Green Liquor, Chemical Recovery. (pp. 332). Elsevier).

The lignin used, according to the present invention, as starting material may have an average molecular weight of 3000-6000 g/mol. The lignin used, according to the present invention, as starting material has a polydispersity of greater than 5. Preferably, the lignin used for the process according to the present invention has an average molecular weight of 3000-6000 g/mol and has a polydispersity of greater than 5.

The process according to the present invention provides lignin oligomers having a molecular weight of 250 to 750 g/mol, preferably 300 to 650 g/mol, more preferably 300 to 500 g/mol and/or having 2 to 10, preferably 2 to 6, more preferably 2 to 4 aromatic moieties. Advantageously, the process according to the present invention may provide low molecular weight lignin oligomers with very uniform size distribution of a polydispersity index (PDI) of below 3.

In one embodiment process according to the present invention provides lignin oligomers with a dispersity between 1 and 3, preferably 1.7 to 2.8, more preferably 1.5 to 2.6, most preferably 2.0 to 2.3.

The process according to the present invention provides lignin oligomers having an oxygen content of less than 20 wt.-% based on the total weight of the lignin oligomers.

Advantageously the catalyst, base, primary alcohol and/or unconverted lignin in the aqueous mixture can be reintroduced in the process for depolymerization of lignin either in a batch or continuous process.

In one embodiment the catalyst and/or the aqueous reaction solution are reintroduced in the process. Reaction solution means the solution obtained after filtration of the precipitated lignin oligomer product. Said reaction solution comprises unreacted or partially depolymerized and partially deoxydehydrated lignin, base, and primary alcohol.

The depolymerized lignin-oligomer produced in the process according to the present invention may be further converted to polymers. In particular, lignin-oligomer produced in the process according to the present invention may be modified chemically or directly used as e.g. cross-linker in different types of polymers. Said polymers may be selected from the group consisting of polyesters, polyurethanes, polyamides and mixtures thereof. The lignin oligomer may be also converted to e.g. biobased aromatics selected from the group consisting of toluene, benzene, xylenes and mixtures thereof.

Disclosed herein is also lignin oligomer composition obtainable by the above described process.

Disclosed herein is also a lignin oligomer composition comprising lignin oligomers, wherein the lignin oligomers have a number average molar mass $M_n$ of 350 to 680 g/mol, preferably 400 to 650 g/mol and a PDI of 1 to 3, preferably 1.7 to 2.8 , more preferably 1.5 to 2.6, most preferably 2.0 to 2.3.

Preferably the O/C weight ratio in the lignin oligomer may be 0.13 to 0.28, preferably 0.15 to 0.21 and optionally the phenolic OH-content may be 1.0 to 2.0 mmol/g lignin oligomer. Preferably, the proportion of the $^1$H-NMR signals of aromatic C-H groups to the aliphatic CH-groups in the lignin oligomer is from 3:1 to 1:8 , more preferably 2:1 to 1:7.5, more preferably from 2:1 to 1:7, most preferably 1:1 to 1:7. The above described lignin oligomer composition may be obtainable or obtained according to the process as described above.

Figures:

Figure 1    shows an overview process flow diagram how to depolymerize and deoxydehydrate lignin according to the invention including optionally recycling steps.

Figure 2    shows IR spectrum of lignin (starting material = native lignin) and lignin oligomers (solid residue = depolymerization product) obtained by the process according to the present invention in Example 1.

Figure 3    shows a diagram of yield of lignin oligomer (solid residue = depolymerization product) yield (wt.-%) over reaction time for Example 1.

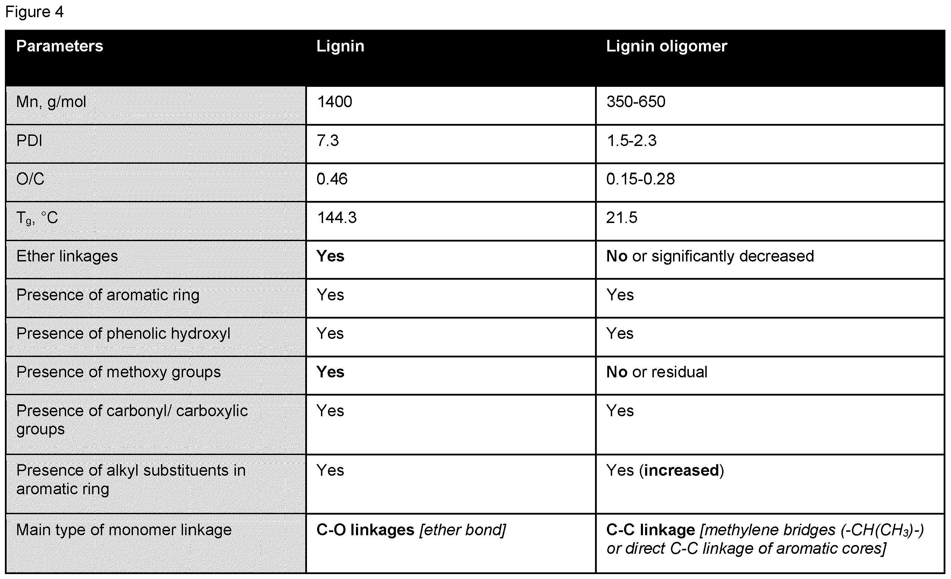

Figure 4    shows a comparison of different parameters of Lignin (Kraft lignin) and the hydrogenated lignin oligomer prepared according to Example 1.

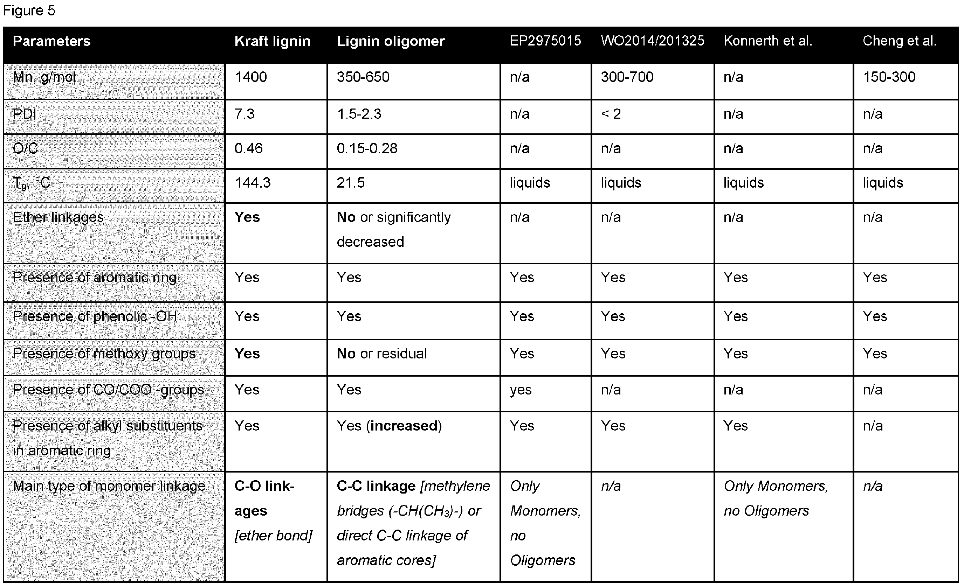

Figure 5    shows a comparison of different parameters of Lignin (Kraft lignin), the hydrogenated lignin oligomer prepared according to Example 1 and hydrogenated lignin material described in the prior art.

Figure 6    shows a $^1$H-NMR of the hydrogenated lignin oligomer obtained by the process according to the present invention in Example 1.

Figure 7    shows a $^1$H-NMR of the lignin starting material used as educt for the process according to the present invention in Example 1.

Figure 8    shows a $^{31}$P-NMR of the hydrogenated lignin oligomer obtained by the process according to the present invention in Example 1.

Figure 9    shows a $^{31}$P-NMR of the lignin starting material used as educt for the process according to the present invention in Example 1.

Figure 10    shows a 2D $^1$H-$^{13}$C HSQC of the hydrogenated lignin oligomer obtained by the process according to the present invention in Example 1.

Figure 11    shows a 2D $^1$H-$^{13}$C HSQC of the lignin starting material used as educt for the process according to the present invention in Example 1.

Figure 12     shows a 2D $^1$H-$^{13}$C HMBC of the hydrogenated lignin oligomer obtained by the process according to the present invention in Example 1.

Figure 13     shows a 2D $^1$H-$^{13}$C HMBC of the lignin starting material used as educt for the process according to the present invention in Example 1.

Examples

General description of experiments:

Kraft-Lignin is prepared as described in US20170355723 and is used as a starting material for depolymerization reaction.

The experiments for lignin depolymerization are carried out at a temperature range of 250-350°C in a 300 ml autoclave. 10 -25 wt.-% of lignin are dissolved in 1N NaOH aqueous solution and put in the autoclave. 0.02-0.26 wt.-% heterogeneous catalyst (Ru/C, Ni Nanoparticles, Cu/Al$_2$O$_3$, Ni/Al$_2$O$_3$) are added. A certain amount of alcohol (MeOH, EtOH, 1-Hexanol, tert-Butyl alcohol, isopropyl alcohol) relative to the NaOH solution is added (0-45 wt.-%). Wt.-% is based on the total weight of the aqueous mixture. The autoclave is flushed with H$_2$ or N$_2$. Then heating is started at a low speed of 80 to 120 rpm to homogenize the temperature. The reaction pressure is adjusted to 90 to 150 bar. After reaching the desired temperature, stirrer speed is adjusted to 2000 rpm. Reaction is performed for 0.5-12 h and then autoclave is cooled down to room temperature, depressurized and solid and liquid parts separated. After dissolution of the solid residue in an appropriate solvent (e.g. EtOH) the catalyst is separated by filtration. The obtained products are analyzed, usually by GPC, elemental analysis and IR-analysis.

GPC-Analysis:

GPC analysis of the lignin used as starting product, the filtrate obtained after reaction as well as the solid residue (the lignin oligomers) after catalyst separation are performed. Samples are dissolved in DMSO+0.5%LiBr as solvent. DMSO+0.5%LiBr is used as eluent as well. The sample is filtered using a 0.2 μm membrane (Sartorius RC 25) before analysis. GPC analysis is performed using Agilent PolarGel M columns in DMSO/LiBr as solvent. Intensities are recorded by a UV-Vis detector (Agilent 1200 VWD 232 nm) and refractive index detector (DRI Agilent 1200 UV). Elution temperature is 35°C, flow rate is 0,5 ml/min. For DMSO+0.5%LiBr SEC calibration is carried out with narrowly distributed polystyrene sulfonate standards from the company PSS with molecular weights of M = 208 to M = 152,000.

Elementary Analysis (EA):

Elementary analysis is performed from freeze-dried samples to remove residual water and solvent. Elementary Analysis is performed using Elementar Microcube machines. One machine is devoted to measure sulfur content and the other one to measure the carbon, nitrogen and hydrogen values. Carbon, hydrogen, nitrogen and sulfur analysis is conducted by combustion followed by thermal conductivity and infrared detection of effluent gases. Sulfur effluent gases are adsorbed in hydrogen peroxide solution and resulting sulfuric acid is titrated with alkali base. The oxygen content was determined by considering proportional formula for organic compounds containing C, H and O.

IR Analysis:

FT-IR spectra of Kraft lignin (starting material) and lignin oligomer (product) were recorded with Alpha-T Transmission FT-IR (MIR) Spectrometer equipped with universal sample module. Solid samples were deposited on a single ZnSe optical window and measured in spectral range of 600-4000 cm-1 in a transition mod.

NMR Analysis:

One-dimensional direct excitation $^1$H and $^{13}$C spectra as well as $^1$H,$^{13}$C-correlation spectra (HSQC, qHSQC, and HMBC) were measured on a Bruker Avance IIIHD 700 spectrometer, operating at 700.30 MHz for $^1$H and 176.10 MHz for $^{13}$C, respectively. For all spectra, a cryogenically cooled (liquid Helium) CPTCI inverse probe was used. Samples were dissolved in DMSO-d6. Measurements were carried out in 5mm NMR tubes.

All $^{31}$P direct excitation spectra were measured on a Bruker Avance III 500 spectrometer, operating at 500.36 MHz for $^1$H and 202.55 MHz for $^{31}$P, respectively. This spectrometer was equipped with a cryogenically cooled (liquid Nitrogen) CPPBBO observe probe. Samples were dissolved in a mixture of CDCl3 and pyridine-d5, as specified in literature. Measurements were carried out in 5mm NMR tubes.

Calculation of polydispersity index (PDI):

Derived from GPC-Data the Mass average molar mass ($M_w$) is measured in [g/mol]

Derived from GPC-Data the Number average molar mass ($M_n$) is measured in [g/mol]

$$PDI = M_w/M_n.$$

Example 1: Standard Experimental conditions

Kraft-Lignin was prepared as described in US20170355723 and was used as a starting material for depolymerization reaction.

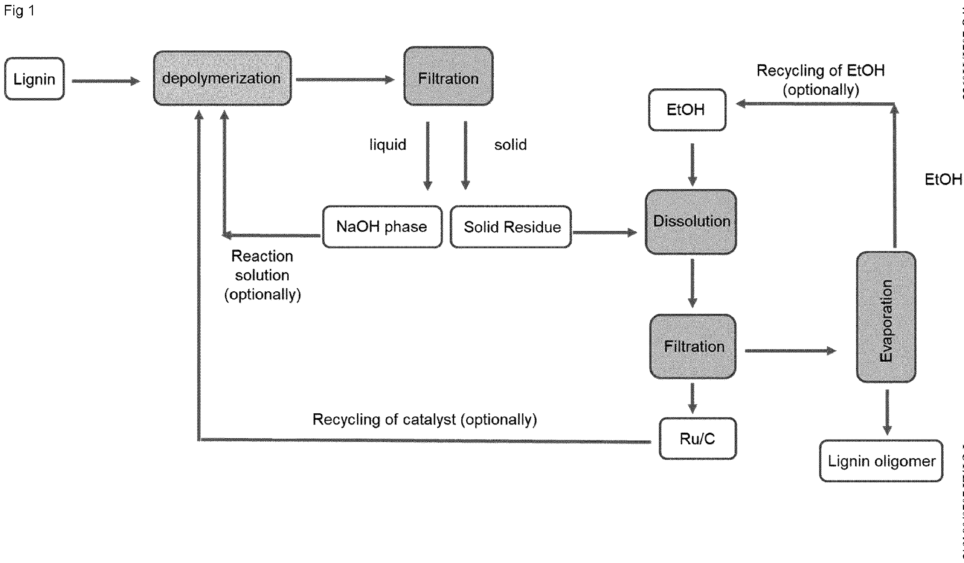

10 g of lignin (10 wt.-%) was dissolved in a mixture of 3.2 g NaOH (3.2 wt.-%) in 76.8 g of deionized water (76.8 wt.-%) and 10 g EtOH (10 wt.-%) solution (final solution pH=13) to obtain an alkaline lignin mixture. Afterwards 260 mg of Ru/C (0.26 wt.-%) catalyst was added to the 10 wt.-% of the alkaline lignin mixture. Wt.-% is based on the total weight of the aqueous mixture. The depolymerization reaction was run for 6 h at 300°C and 120 bar in $H_2$-atmosphere. Lignin depolymerization resulted in a solid residue and an aqueous reaction solution. Workup was done by depressurizing the reaction mixture at room temperature, filtering off the reaction solution. The remaining solid residue (lignin oligomer) was dissolved in EtOH. The catalyst that was trapped in the solid residue does not dissolve in EtOH and could be filtered off and recycled for the next experiment. (see Fig. 1). Both catalyst-free solution of lignin-oligomer and the filtrated reaction solution were analyzed by GPC (see Table 1).

Table 1 Molar mass and PDI of Kraft lignin (starting material), solid residue (lignin oligomer) and filtrated reaction solution determined by GPC analysis

| | $M_n$, g/mol | $M_w$, g/mol | PDI=$M_w/M_n$ |
|---|---|---|---|
| Kraft lignin (starting material) | 1400 | 10200 | 7.3 |
| solid residue = lignin oligo-mer | 581 | 1410 | 2.2 |
| filtrated reaction solution | 615 | 1330 | 2.4 |

Molecular masses and PDI were determined by DMSO+LiBr GPC using polystyrene sulfonate calibration

Formation of 5.9 g (59 % yield) of solid residue (lignin oligomer) in the bottom of the autoclave was noticed. Obtained solid residue was well soluble in ethanol, acetone, THF, DMSO (except catalyst) and not soluble in water, toluene. Solubilization of solid residue in ethanol with subsequent filtration of Ru/C catalyst resulted in solution of lignin oligomer (see Fig.1). No formation of insoluble char was found under applied conditions. The dissolved solid residue (lignin oligomer solution) could be dried and re-dissolved in ethanol.

The solid residue and reaction solution (the filtrate) were analyzed by GPC, elemental analysis and IR analysis.

Product characterization:

GPC performed in DMSO+LiBr as solvent and eluent:

- Lignin (starting material)
- Solid residue, re-dissolved in EtOH (catalyst filtered off) = lignin oligomer solution
- Reaction solution (Filtrate, not yet depolymerized lignin, NaOH, EtOH solution) shows distinct molecular weight characteristics:
- Starting Lignin: Average molecular weight of 3400 g/mol and broad dispersity PDI=7.3.
- Solid residue = lignin oligomer: Average molecular weight of 540 g/mol and a narrow dispersity around PDI= 2, preferably 2.3.
  This material is fully soluble in EtOH; no traces of e.g. char-like substances are left in the reaction vessel after rinsing with EtOH.
- Reaction solution (Filtrate): Lower molecular weight distribution than in starting material but still high oxygen content (see Tables 1 and 3).

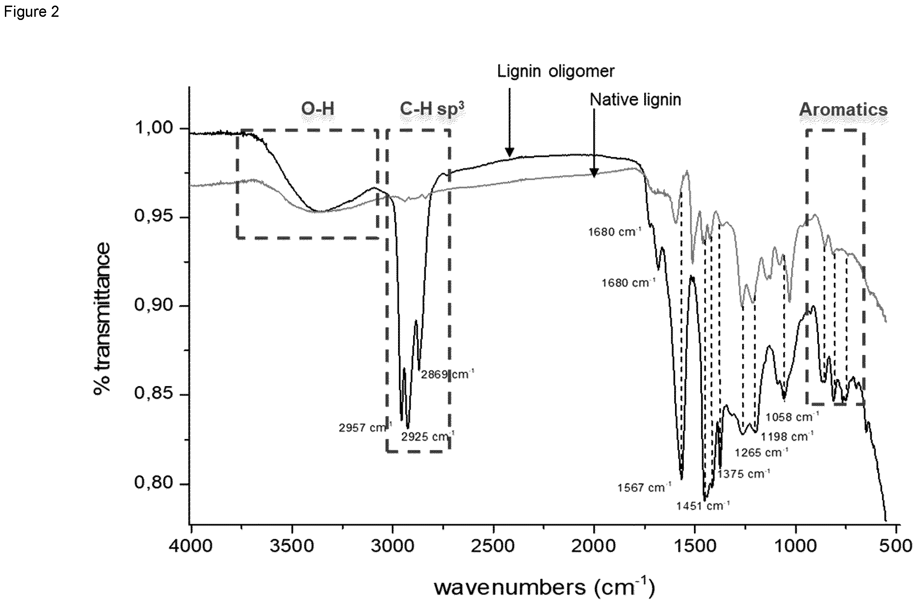

IR analysis of Solid residue, i.e. lignin oligomer shows similar aromatic bands to starting lignin and additionally a strong increase of aliphatic CH-signals and free aromatic OH bands (Figure 2). Characteristic bands associated with lignin are summarized in Table 2. The bands related to aromatics C-H out of plane bending at 700-900 cm$^{-1}$ and O-H stretching of hydroxyls more likely related to phenolic groups at 3000-3500 cm$^{-1}$ are both present in starting lignin and lignin oligomer. The presence of three different bands for C-H out of plane bending at 867, 860, 811, 766, 750 cm$^{-1}$ reveal that different substituents present in the aromatic rings. In particular, presence of 867 and 860 cm$^{-1}$ band are associated with ortho- and para-substituted aromatics.

Table 2 Characteristic bands of lignin and lignin oligomer*

| $1/\lambda$, cm$^{-1}$ | Absorption/vibration | Functional group |
| --- | --- | --- |
| 3390 | -O-H stretching * | -O-H, hydrogen bonds |
| 2940 | -C-H stretching* | -CH$_2$-; -CH-; -CH$_3$ |
| 1702 | C=O stretching | Carbonyl groups |
| 1595; 1514 | C=C stretching | Aromatic C=C bonds (benzene ring) |
| 1455; 1422 | C-H deformation* | Alkanes, -CH$_2$- |
| 1260-1084 | C-O stretching; typical vibration for sulfur compounds* | -C-O-; -C=S; -SO$_2$- |
| 1030 | C-O stretching; -CH$_2$- bending | -C-O-; -CH$_2$- |
| 855; 814 | C-O stretching; -CH$_2$- bending* | 1,4-substitution; 1,3,4-trisubstitu-tion of benzene ring |
| *Additional bands for depolymerized lignin | | |

Table 3 shows elemental analysis of starting material (lignin) and the solid residue (lignin oligomers) obtained by the process according to the present invention and the not yet incompletely depolymerized and deoxydehydrated lignin fraction (filtrated reaction solution) that is still soluble in the aqueous reaction mixture (the filtrate).

Table 3 Elemental composition of Kraft lignin (starting material), solid residue and filtrated reaction.

| Sample | C [%] | O [%] | H [%] | O/C |
| --- | --- | --- | --- | --- |
| Kraft lignin (starting material) | 64.3 | 29.7 | 6 | 0.46 |
| filtrated reaction solution | 57.5 | 35.7 | 6.8 | 0.55 |
| solid residue = lignin oligomer | 78.7 | 13.3 | 8 | 0.17 |

In comparison with the native lignin that contains 64.3 wt.-% of C, 29.7 wt.-% of O and 6.0 wt.-% of H the depolymerized product (lignin oligomer) exhibits significant deoxydehydration giving 13.3 wt. % of O. In parallel, the quantity of H and C increased to 8 wt.-% and 78.7 wt.-% respectively. The decrease of oxygen content is attributed to the deoxydehydration reaction catalyzed by e.g. Ru/C catalyst.

Example 2: Effect of alcohol addition

To evaluate the effect of alcohol these additional experiments were performed. The standard experiment (Example 1) was compared to the same experiment performed only in NaOH (no alcohol addition) and compared to an experiment performed with higher amounts EtOH content (NaOH:EtOH = 1.8:45, wt.%/wt.% based on the total weight of the aqueous mixture) and smaller amounts EtOH content (NaOH:EtOH=3.6:5, wt.%/wt.% based on the total weight of the aqueous mixture).

In experiments with low or no alcohol addition no soluble solid residue was found. In pure NaOH medium char-like residue was formed. This char-like solid residue was completely insoluble in solvents like in ethanol, acetone, THF, DMSO, water and/or toluene.

In the experiment with higher EtOH content the nature of obtained solid residue=lignin oligomers is similar to the standard condition based on elemental analysis (Table 5). Both products have O/C ratio in the range of 0.16-0.17 that is significantly different from starting lignin material where O/C ratio is 0.46. However, the workup is more difficult as lower temperature below 5°C is required for better recovery of lignin oligomer product by precipitation. Thus, addition of alcohol prevents insoluble char-like residue formation but should not be too high to allow efficient product precipitation and recovery. The results of alcohol addition and characteristics of obtained products are summarized in Tables 4 and 5.

Table 4 Experiments performed with different amounts of EtOH

| Alcohol | NaOH (aq) | Lignin con-tent | Ru/C | Soluble Lignin-Oli- gomers | Char-coal formation |
| --- | --- | --- | --- | --- | --- |
| No | 90 wt.-% | 10 wt.-% | 0.26 wt.-% | no | yes |
| 10 wt.- %EtOH | 80 wt.-% | 10 wt.-% | 0.26 wt.-% | yes | no |
| 45 wt.- %EtOH | 45 wt.-% | 10 wt.-% | 0.26 wt.-% | yes | no |
| 5 wt.- %EtOH | 90 wt.-% | 5 wt.-% | 0.13 wt.-% | no | yes |

Table 5 Elemental analysis of solid residue (soluble lignin oligomers) obtained under standard conditions using 10 and 45 wt% of EtOH

| Alcohol | NaOH (aq) | C % | O % | S % | N % | H % | O/C |
| --- | --- | --- | --- | --- | --- | --- | --- |
| 10 wt% EtOH | 80wt.-% | 74.5 | 12.3 | 0.15 | <0.5 | 8 | 0.17 |
| 45 wt% EtOH | 45wt.-% | 78.8 | 12.3 | 0.13 | <0.5 | 8.4 | 0.16 |

Example 3: Effect of alcohol

To determine the effect of alcohol type the lignin depolymerization was performed as described in Example 1 and additionally with MeOH, 2-Propanol, t-BuOH or n-Hexanol instead of EtOH. Results are shown in Table 6. Lignin oligomer product formation was noticed for the reaction performed in MeOH and EtOH. In case of MeOH the yield of product was 18 % while for EtOH the yield was 59 %. In addition, product received in case of performing depolymerization in the presence of MeOH had slightly higher O/C ratio, i.e. lower degree of deoxydehydration (Table 7). No product formation was noticed for the reaction performed with 2-Propanol, t-BuOH and n-Hexanol. The recovered liquid phase was freeze-dried and further analyzed by EA.

Table 6 Influence of alcohol nature on the lignin oligomer formation

| Additive | NaOH (aq) content | Lignin content | Lignin Oligomer |
| --- | --- | --- | --- |
| 10 wt% EtOH | 80 wt.-% | 10 wt.-% | yes (59% yield) |
| 10 wt% MeOH | 80 wt.-% | 10 wt.-% | yes (18 % yield) |
| 10 wt% 2-Propanol | 80 wt.-% | 10 wt.-% | no |
| 10 wt% t-BuOH | 80 wt.-% | 10 wt.-% | no |
| 10 wt% n-Hexanol | 80 wt.-% | 10 wt.-% | no |
| Yield means: mass ratio of starting material (lignin) to dried lignin oligomer (product) | | | |

Table 7 Elemental analysis of lignin oligomer products using ethanol and methanol as an additive

| Alcohol | C % | O % | S % | N % | H % | O/C |
| --- | --- | --- | --- | --- | --- | --- |
| EtOH | 74.5 | 12.3 | 0.15 | <0.5 | 8 | 0.17 |
| MeOH | 72 | 17.2 | 0.15 | <0.5 | 7.1 | 0.24 |

Example 4: Effect of catalyst addition

To determine the effect of heterogeneous catalyst the lignin depolymerization was performed without catalyst addition using EtOH or MeOH. Lignin depolymerization was performed under standard conditions at 300°C for 6 h and 120 bar as described in Example 1 but without catalyst addition using two different alcohols (EtOH or MeOH) and two different NaOH:alcohol ratios (3.2:10 or 3.6:5, wt.%/wt.% based on the total weight of the aqueous mixture). No lignin oligomer product formation was noticed for the reaction performed without catalyst. However, some solvent-insoluble char-like residue was found. The liquid phase was freeze-dried and further analyzed by Elemental analysis. Elemental analysis reveals that nature of product obtained without catalyst addition is completely different. Indeed, all reaction products obtained without catalyst addition have high oxygen content in the range of 31-35 wt.-% which is comparable to the starting lignin (37.4 wt.-%). The calculated O/C ratios for the products varies in the range of 0.60-0.68. In contrary depolymerization performed in the presence of a catalyst results in significant decrease of oxygen content till 12-14 wt. % that is results in 0.17 O/C ratio (Table 8). This shows that a deoxydehydration catalyst is required to obtain lignin oligomers with a molecular weight (Mw) below ~ 500 and a uniform dispersity (PDI~2). Thus, performing lignin depolymerization without a catalyst does not lead to the desired product and results in lignin oligomers with high dispersity.

Table 8 Comparison of lignin depolymerization in the presence and in the absence of Ru/C catalyst

| Catalyst | NaOH (aq) content | Alcohol content | Lignin content | Lignin Oligomer | Insoluble char | O/C |
|---|---|---|---|---|---|---|
| **Ru/C** | 80 wt.-% | 10 wt.-% EtOH | 10 wt.-% | yes | no | 0.17 |
| - | 80 wt.-% | 10 wt.-% EtOH | 10 wt.-% | no | yes | 0.61 |
| - | 80 wt.-% | 10 wt.-% MeOH | 10 wt.-% | no | yes | 0.60 |
| - | 90 wt.-% | 5 wt.-% EtOH | 5 wt.-% | no | yes | 0.69 |
| - | 90 wt.-% | 5 wt.-% MeOH | 5 wt.-% | no | yes | 0.68 |

Example 5: Using other catalysts

Other catalysts, like Ni nanoparticles, $Ni/Al_2O_3/SiO_2$ and $Cu/Al_2O_3/SiO_2$ were tested for lignin depolymerization under standard conditions as described in Example 1 and analyzed by GCP (Table 9) and elemental analysis (Table 10). In all cases, the soluble solid residue was found after the reaction (standard reaction conditions: 6h, 300°C, 120bar). However, in case of using Ni Nanoparticles (NPs) the depolymerized product had higher polydispersity (PDI=2.8,) and slightly higher mass ($M_n$=591 g/mol) in comparison with Ru/C (PDI=2.3, $M_n$=508 g/mol) (see Table 7). The biggest difference was found in elemental analysis of obtained product. In case of using Ni NPs the amount of oxygen was higher than for Ru/C (see Table 10). Ni on alumina silica catalyst resulted in product with the same characteristics as the product received under standard conditions with Ru/C in terms of molar masses, dispersity and O/C ratio. However, the yield in this case was only 24% that is two times less than in case of Ru/C catalyst while the loading of Ni was ten times bigger in comparison with Ru. Application of Cu on alumina silica support however resulted in the lowest molar masses of depolymerized lignin ($M_n$=473 g/mol) and dispersity (PDI=1.7). Using Cu on alumina silica support also resulted in 43% yield that is just 16% less than by using more expensive Ru/C catalyst. The loading Cu in this case was six times bigger than Ru in used in standard conditions. GPC chromatogram of product obtained by applying different catalyst are shown in the Table 9.

Table 9 Characteristics of obtained lignin oligomer product by using different catalysts

| Catalyst | Het. Cat. loading | Metal loading, wt% | $M_n$, g*mol⁻ | $M_w$, g*mol⁻¹ | PDI | Yield of Lignin Oligomer |
|---|---|---|---|---|---|---|
| **Ru/C** | 0.26 wt.-% | 0.01 | 508 | 1160 | 2.3 | 59% |
| **Ni NPs** | 0.02 wt.-% | 0.02 | 591 | 1640 | 2.8 | 48%* |
| **$Ni/Al_2O_3/SiO_2$** | 0.26 wt.-% | 0.12 | 562 | 1360 | 2.4 | 24% |
| **$Cu/Al_2O_3/SiO_2$** | 0.26 wt.-% | 0.06 | 473 | 789 | 1.7 | 43% |
| Molecular masses and PDI were determined by DMSO+LiBr GPC using polystyrene sulfonate calibration Yield means: mass ratio of starting material (lignin) to dried lignin oligomer (product) | | | | | | |

Table 10 Elemental analysis data of product obtained by using different catalysts

| Sample | C % | O % | S % | N % | H % | O/C |
|---|---|---|---|---|---|---|
| Ru/C | 74.5 | 12.3 | 0.15 | <0.5 | 8 | 0.17 |
| Ni NPs | 68.1 | 19.5 | 0.22 | <0,5 | 8.1 | 0.28 |
| $Ni/Al_2O_3/SiO_2$ | 77 | 11.7 | 0.25 | <0.5 | 7.4 | 0.15 |
| $Cu/Al_2O_3/SiO_2$ | 75.1 | 12.3 | 0.21 | <0.5 | 7.4 | 0.16 |

Example 6: Effect of gas nature and pressure

To analyse the effect of the gas nature and pressure lignin depolymerization was performed under standard conditions (NaOH:EtOH= 3.2:10 (wt.%/wt.%), 10 wt.-% Lignin, 2.6 wt.-% of Ru/C, 300°C, 6 h) using 120 bar reaction pressure in $H_2$ atmosphere as described in Example 1. Substitution of $H_2$ with $N_2$ also resulted in formation of a soluble solid residue (lignin oligomer). The solid residue was re-dissolved in EtOH and filtrated to remove Ru/C catalyst. Obtained products were analyzed by GPC and Elementary analysis. The GPC chromatogram of reaction product obtained with $N_2$ was very similar to the standard conditions where 120 bar of $H_2$ were applied (see Table 11) and resulted in low dispersity (PDI=2.3) and molar mass ($M_n$=550 g/mol). However, decrease of pressure from 120 bar to 90 bar led to lower degree of depolymerization that resulted in slightly higher dispersity (PDI=2.5) and higher molar mass of residue ($M_n$=678 g/mol). Performing depolymerization with $N_2$ instead of $H_2$ led to the higher yields of solid residue (product). The characteristics of obtained products were summarized in Table 11. Elementary analysis reveals similar nature of obtained products (see Table 12). A slight difference in composition was found in case of performing depolymerization under 90 bar of $N_2$. No nitrogen incorporation into polymer structure was found for experiments performed under $N_2$ pressure.

Table 11 Influence of gas nature and pressure on the residues found

| Non-Oxidizing gas. | Reaction pressure at . 300°C | $M_n$, g*mol$^{-1}$ | $M_W$, g*mol$^{-1}$ | PDI | Yield of lignin oligomer |
|---|---|---|---|---|---|
| H2 | 120 bar | 508 | 1160 | 2.3 | 59% |
| N2 | 120 bar | 550 | 1290 | 2.3 | 70% |
| N2 | 90 bar | 678 | 1 680 | 2.5 | 66% |
| $N_2$+$H_2$(1:1) | 120 bar | 542 | 1270 | 2.4 | 41% |
| Molecular masses and PDI were determined by DMSO+LiBr GPC using polystyrene sulfonate calibration Yield means: mass ratio of starting material (lignin) to dried lignin oligomer (product) | | | | | |

Table 12 Effect of gas nature and pressure on elemental composition of obtained products

| Non-oxidizing gas | Reaction pressure at 300°C | C % | O % | S % | N % | H % | O/C |
|---|---|---|---|---|---|---|---|
| $H_2$ | 120 bar | 74.5 | 12.3 | 0.15 | <0.5 | 8 | 0.17 |
| $N_2$ | 120 bar | 74.5 | 13.3 | 0.12 | <0.5 | 7.7 | 0.18 |
| $N_2$ | 90 bar | 75.5 | 14 | 0.24 | <0.5 | 7.3 | 0.18 |
| $N_2$+ H2(50%/50%) | 120 bar | 76 | 12.4 | 0.14 | <0.5 | 8 | 0.16 |

Example 7: Effect of reaction time

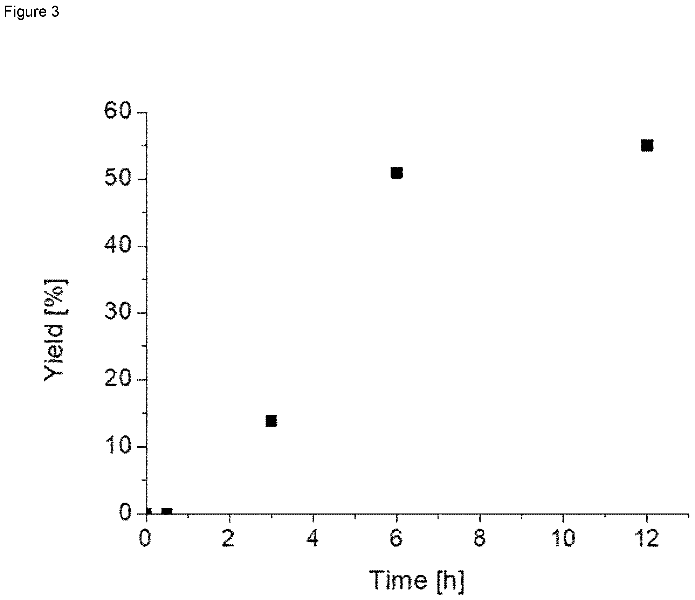

To determine the effect of reaction time on product formation reaction time was monitored and product formation in Example 1 was analyzed by GPC and elementary analysis. No lignin oligomer precipitate was found within 0.5 h of depolymerization and 14 wt.-% lignin oligomer precipitate formed within 3 h (see Fig.3). Performing reaction for 6 h delivered significant yield of lignin oligomer precipitate (51 wt.-%). However, increase of depolymerization time to 12 h lead to slight additional product yield of 55 wt.-%. Comparison of GPC chromatogram and elementary analysis of products obtained after 3, 6 and 12 h show same product characteristics (see Fig. 3 (Yield over time) & Tables 13/14). In addition, longer reaction time leads to decrease in Sulphur content. 5 h to 7 h reaction time are sufficient for obtaining desired product in high yield.

Table 13 Product PDI and yield depending on reaction time

| Reaction time | $M_n$, g/mol | $M_w$, g/mol | PDI | Yield |
|---|---|---|---|---|
| 3 h | 499 | 1080 | 2.2 | 14 % |
| 6 h | 508 | 1160 | 2.3 | 51 % |
| 12 h | 489 | 886 | 1.8 | 55 % |
| Yield means: mass ratio of starting material (lignin) to dried lignin oligomer (product) | | | | |

Table 14 Comparison of elemental analysis of starting lignin with lignin depolymerized products at different time intervals

| Sample | C % | O % | S % | N % | H % |
|---|---|---|---|---|---|
| Starting lignin | 61.9 | 28.5 | 1.8 | >0.5 | 5.8 |
| 0.5 h | No precipitate formed | | | | |
| 3h | 76.6 | 12.3 | 0.16 | <0.5 | 8.1 |
| 6h | 74 | 14.2 | 0.22 | <0.5 | 7.6 |
| 12 h | 74.3 | 12.5 | 0.11 | <0.5 | 8.3 |

Example 8: Effect of temperature

To determine the effect of applied temperature three experiments at 250°C, 280°C, 300°C and 335°C as described in Example 1 were performed using standard depolymerization conditions. No lignin oligomer precipitate was found after performing reaction at 250°C for 6 h. Thus, temperature >250°C is required for product formation within 6 h of reaction time. Increasing temperature to 335°C resulted in formation of insoluble char (not the desired lignin oligomer). Thus, for optimized product formation and reduced or no char formation, reaction temperatures between 250 and 320°C, preferably 280°C to 320°C, are recommended (see Table 15).

Table 15 Effect of temperature on formation of lignin oligomer and char

| Temperature | Lignin Oligomer | Insoluble char |
|---|---|---|
| 250 °C | no | no |
| 280 °C | yes | no |
| 300 °C | yes | no |
| 320 °C | yes | no |
| 335°C | yes | yes |

Example 9: Effect of base concentration

The impact of base concentration was studied by comparing lignin depolymerization using 1.0 M NaOH (NaOH:EtOH=3.2:10, wt.%/wt.% based on the total weight of the aqueous reaction solution) and using 0.3 M NaOH (NaOH:EtOH=1:10, wt.%/wt.% based on the total weight of the aqueous reaction solution)). All other conditions were same as in standard Example 1. In both cases lignin oligomer was formed. Elemental analysis revealed similar composition of both products and identical O/C ratio (see Table 16). Both products possessed similar molar masses between 450-550 g/ mol and dispersity in the range of 1.8-2.7. The yield of product obtained using 0.3 M NaOH was 6.0 g compared to 5.9 g of product obtained with 1.0 M NaOH.

Table 16 Elemental analysis of tar-like precipitated products obtained under standard conditions using 1.0 M NaOH and 0.3 M NaOH

| Base | C % | O % | S % | N % | H % | O/C |
|---|---|---|---|---|---|---|
| 1.0 M NaOH | 74.5 | 12.3 | 0.15 | <0.5 | 8 | 0.17 |
| 0.3 M NaOH | 76.8 | 13.5 | 0.21 | <0.5 | 7.9 | 0.17 |

Example 10: Effect of lignin loading

To determine the effect of lignin loading on product formation of lignin oligomer, the depolymerization under standard conditions (10 wt.-% of lignin using NaOH:EtOH=3.2:10 wt.%/wt.% based on the total weight of the aqueous reaction solution) was compared with a reaction using double amount of lignin (20 wt.-% of lignin, NaOH:EtOH = 2.8:10 wt.%/wt.% based on the total weight of the aqueous reaction solution). An increased yield of solid residue product from 59% for standard experiment with 10 wt.-% lignin content to 76 % in case of 20 wt.-% lignin content was observed. The yield was calculated by (weight of product/weight of starting lignin)*100%. All other conditions were same as in standard Example 1.

The product was dissolved in EtOH and characterized by GPC and elemental analysis. Both products exhibited similar elemental composition.

Example 11: Recycling

A recycling experiments was done by applying 90 bar reaction pressure in N2-Atmosphere, the other reaction conditions were kept constant (see Example 1).

The reaction solution (filtrate) obtained after first cycle of lignin conversion, together with recovered Ru/C catalyst were re-used for a second cycle. In this case a new portion of lignin equal to the mass of recovered product in the first catalytic cycle was added. Thus, concentration of lignin was kept constant in first and second cycles. The reaction solution, containing the base and the alcohol and not yet precipitated lignin was recycled as well.

In both cycles similar yields of residue were obtained. Characteristics of obtained products are summarized in the Table 17.

Table 17 Comparison of products obtained in I and II cycles under recycling conditions described in method B

| Sample | $M_n$, g*mol$^{-1}$ | $M_w$, g*mol$^{-1}$ | PDI | O/C | Yield Lignin Oligomer |
|---|---|---|---|---|---|
| I cycle | 678 | 1680 | 2.5 | 0.18 | 70% |
| II cycle | 504 | 1060 | 2.1 | 0.14 | 66% |
| Molecular masses and PDI were determined by DMSO+LiBr GPC using polystyrene sulfonate calibration | | | | | |

The recycling procedure showed that Ru/C catalyst, the reaction solution comprising alcohol, base and residual lignin can be recycled. A schematic recycling procedure is presented in Figure 1.

Example 12 ($^1$H-NMR spectra of the lignin oligomer obtained according to example 1 and the lignin starting material):

155 mg/ml sample (Lignin or lignin oligomer) were dissolved in deuterated DMSO. The Sample was fully soluble.

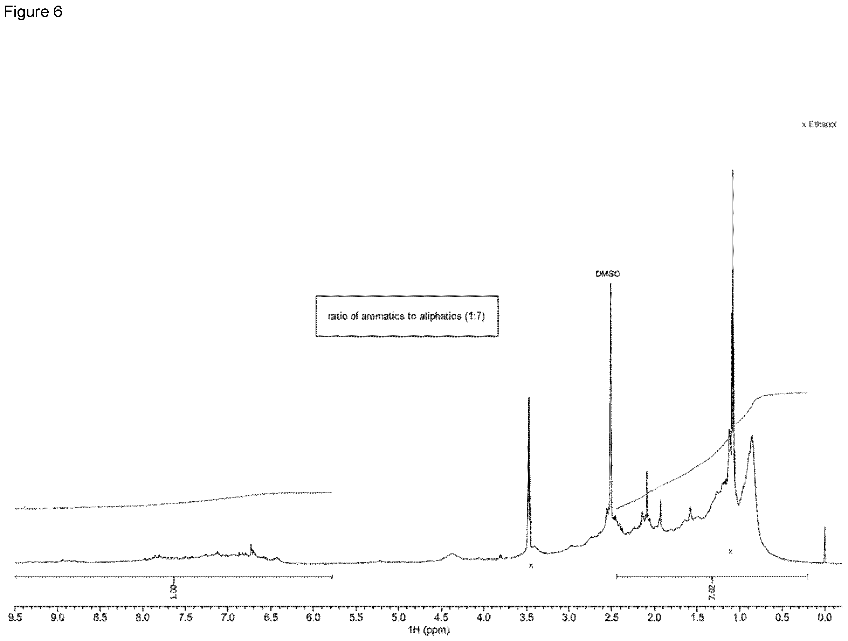

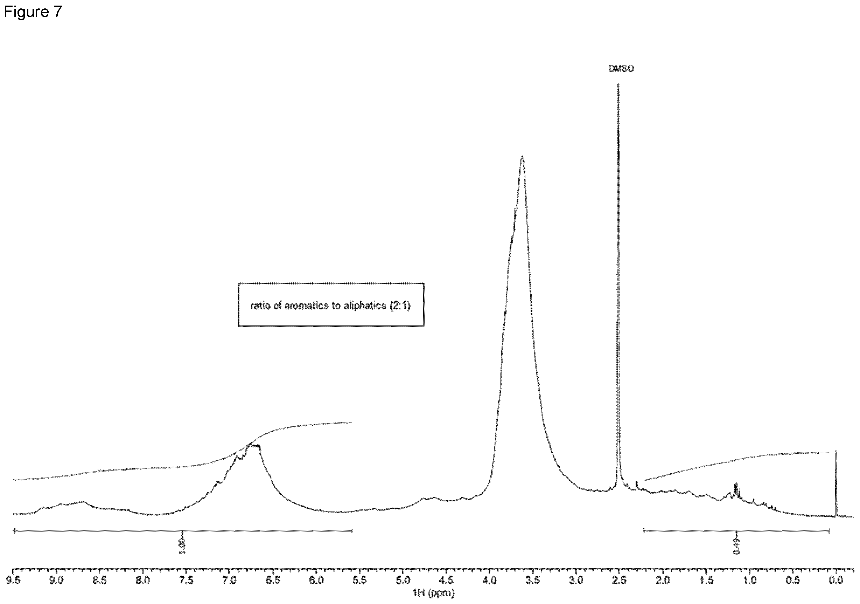

Comparing the 1H-NMR spectra of the lignin oligomer (obtained according to example 1) relative to the starting lignin material shows less aromatic signals in the hydrogenated lignin oligomer sample. The proportion of C-H-aromatics signals to the C-H- aliphatic signals in the lignin oligomer sample is 1:7 (Figure 6) compared to 2:1 in the lignin starting material (Figure 7). In the range of 3.5 to 4 ppm a strong decrease of the $CH_2$-OH and Aromatic-O-$CH_3$ is observed due to hydrogenation of the lignin oligomer. Exception is a quartett-signal, that can be attributed to residual reaction solvent Ethanol, which was not completely removed from the lignin oligomers. The corresponding triplett in the aliphatic region of the spectrum increases the integral over the aliphatic region relative to the aromatic region.

Example 13 ($^{31}$P-NMR spectra of the lignin oligomer obtained according to example 1 and the lignin starting material):

$^{31}$P-NMR Analysis: 15 mg sample (lignin or lignin oligomer) were treated w/ 50 μl 2-Chlor-4,4,5,5-tetramehtyl-1,3,2-dioxaphospholan in 400 $\mu$l Pyridine and CDCl3 (1,6:1 v/v). Additionally, cyclohexanol as internal standard and Cr(III)-Acetylacetonat (as relaxation agent) were added in 150 $\mu$l Pyridine/CDCl3. The sample was analyzed as "quantitative 31P-NMR".

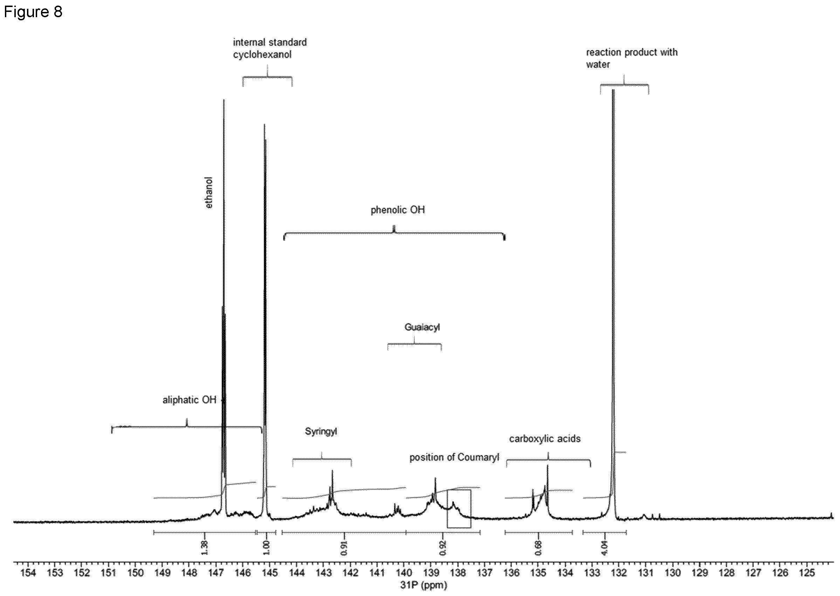

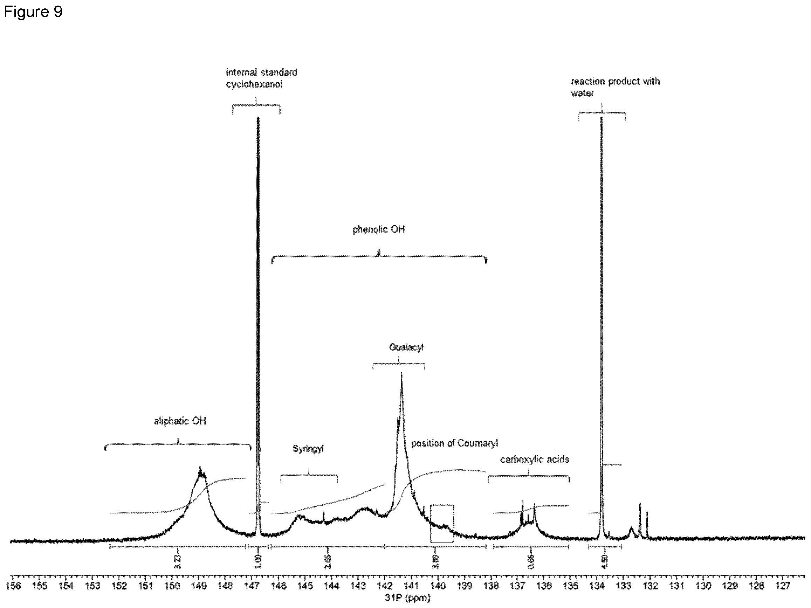

The $^{31}$P-NMR signals were attributed according to literature: J. Agric. Food Chem. Vol. 43, No. 6, 1995 - Granata and Argyropoulos). The sample shows signals for aliphatic-OH, phenolic-OH and carboxylic acids. Syringyl. And Guaiacyl-rings can be distinguished. The total amount of aliphatic, phenoic and carboxylic OH were determined. Table 18 shows the differences in the signals between lignin (starting material, Figure 9) and lignin oligomer (product, Figure 8).

Tabelle 18 Calculation $^{31}$P-NMR

| | beta [mg/mg] | M [g/mol] | V [$\mu$l] | m [mg] in 150 $\mu$l | N [mmol] | | |
|---|---|---|---|---|---|---|---|
| Cyclohexanol | 6.38 | 100.158 | 150 | 0.957 | 0.009554903 | | |
| Cr(III) acac | 5.8 | | | | | | |
| Results | Sample M [mg] | OH-Content in [mmol/g] | | | | | |
| | | Aliph.-OH | Syringyl and phenolic OH | Guaiacyl | Carboxyl-OH | Total phenolic OH | Total -OH |
| Lignin Oligomer | 14.16 | 0.93 | 0.61 | 0.62 | 0.46 | 1.23 | 2.62 |
| Lignin | 15.03 | 1.54 | 1.26 | 1.86 | 0.32 | 3.12 | 4.98 |

Example 14 (2D 1H-13C HSQC NMR spectra of the lignin oligomer obtained according to example 1 and the lignin starting material):

155 mg/ml sample (lignin or lignin oligomer) were dissolved in deuterated DMSO. The Sample was fully soluble.

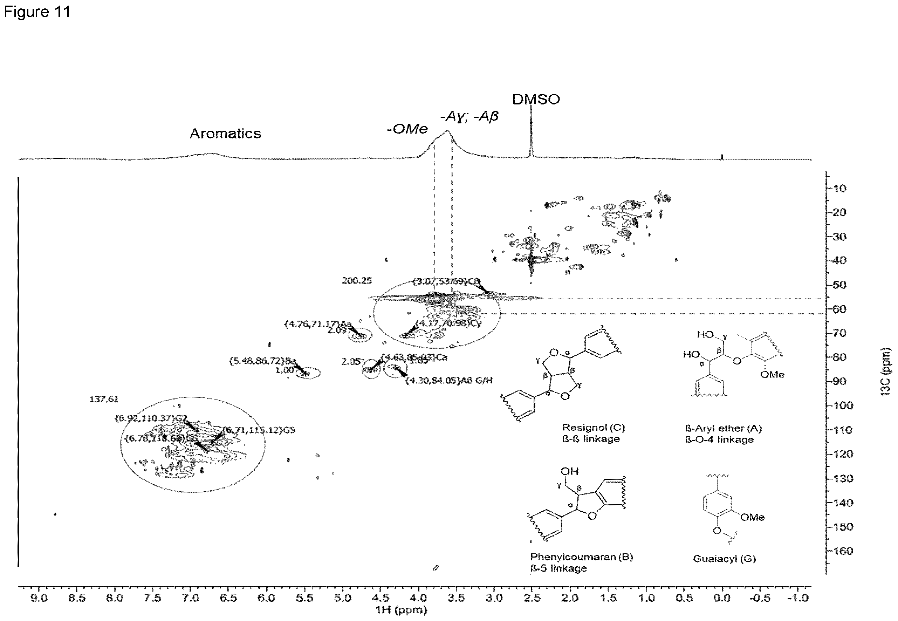

2D $^1$H-$^{13}$C HSQC NMR of initial Kraft lignin (Figure 11) shows the aromatic protons of the monomer units by cross-correlation peaks at $\delta_C/\delta_H$ 110-118/6.7-6.9 ppm. These units are linked in a 3D polymer network by different binding motifs which the $\beta$-O-4 linkage (A) and is most pronounced. The cross peaks at $\delta_C/\delta_H$ 60-65/3.2-3.7 ppm are attributed to the protons linked to $\beta$ and $\gamma$ carbons in (A). Other structures like resignol (C) (series of cross peaks at $\delta_C/\delta_H$ 85.03/4.63, 53.69/3.07 and 70.98/4.17 attributed to protons linked to $\alpha$, $\beta$ and $\gamma$ carbons accordingly) and phenylcumaran (B) ($\delta_C/\delta_H$ 86.72/5.48 attributed to proton at $\alpha$ position) were detected as well. The integration of protons attached to $\alpha$ carbon in each structure allowed to calculate the relative ratio of linkages: 42 % of A ($\beta$-O-4 linkage), 37 % of C ($\beta$-$\beta$ linkage) and 21 % of B ($\beta$-5 linkage).

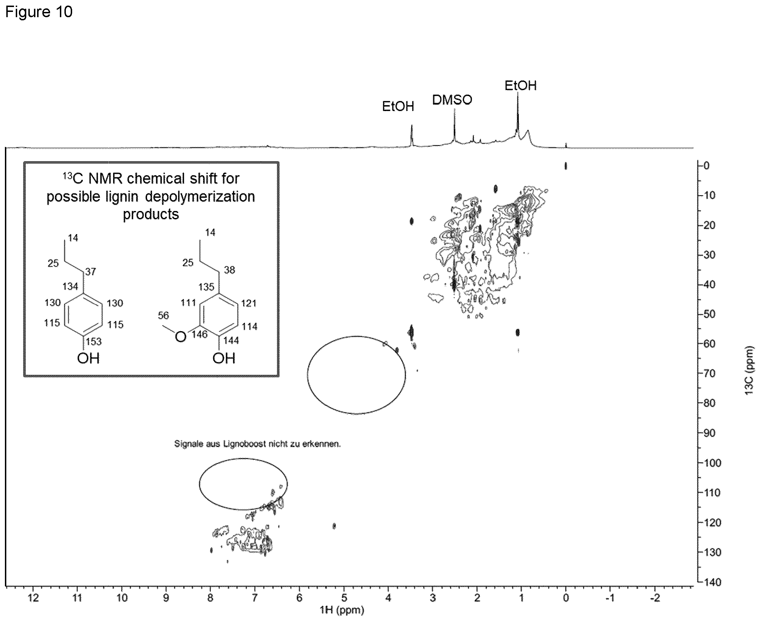

2D $^1$H-$^{13}$C HSQC NMR_of 'Lignin oligomer' product (Figure 10) shows significant difference in comparison to the starting Kraft lignin. A major difference is the absence of x-correlation peaks in the region of $\delta_C/\delta_H$ 50-90/3.0-4.5 ppm. These signals in Kraft-Lignin were associated to $\beta$-O-4, $\beta$-$\beta$ and $\beta$-5 linkages. This indicates an almost quantitative cleavage of ether linkages under the applied reaction conditions and proving the lignin depolymerization. Additionally, the signal associated with -$OCH_3$ group of aromatic cores ($\delta_C/\delta_H$ 56/3.8 ppm) is absent as well.

More considerable differences were found in the aromatic regions. The starting Kraft lignin (Fig.1) displayed aromatics signals in the region of $\delta_C/\delta_H$ 105-120/6.2-7.7, caused by the presence of electron withdrawing methoxy groups at the aromatic cores. The aromatic signals of the lignin oligomer are instead shifted to $\delta_C/\delta_H$ 120-130/6.8-8.0, which is caused by the absence of electron withdrawing -$OCH_3$ groups attached to aromatic cores. Comparison of chemical shifts of the obtained lignin-oligomer and monomers resembling lignin repeating units (e.g. 4-propylphenol and 2-methoxy-4-propylphenol, inset in Fig 10) revealed significant similarity. The shifts of 4-propylphenol seem to fit best to the obtained structure.

The strong correlation at 55 ppm in $^{13}$C spectrum and 3.5-4.5 ppm in $^1$H spectra is related to the -$OCH_3$ groups of the aromatic cores. Alkyl-chains, resulting from the p-propyl-group are found at $\delta_C/\delta_H$ 13-45/0.8-3.0 ppm.

Example 15 (2D $^1$H-$^{13}$C HMBC NMR spectra of the lignin oligomer obtained according to example 1 and the lignin starting material):

2D $^1$H-$^{13}$C HMBC NMR 155 mg/ml sample (lignin or lignin oligomer) were dissolved in deuterated DMSO. The Sample was fully soluble.

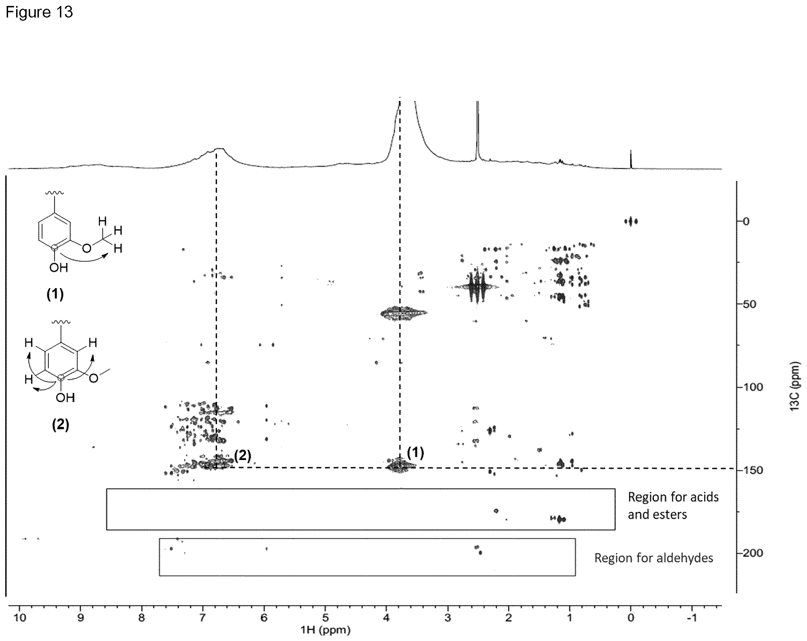

2D $^1$H-$^{13}$C HMBC NMR of Kraft lignin (Figure 13) possess a high number of cross-correlation peaks due to the complex structure of the polymer. The most pronounced correlation are at δC/δH 150/6.5-7.5 ppm (2) which are assigned to aromatic protons in proximity to quaternary, heteroatom bound aromatic carbon (CH-C(OH)-CH). This proves the presence of phenolic groups in the polymer structure. The cross peaks δC/δH 150/6.5-3.9 ppm result from correlation of protons of--OCH$_3$ group with quaternary, heteroatom bound aromatic carbon (CH-C(OH)-CH).

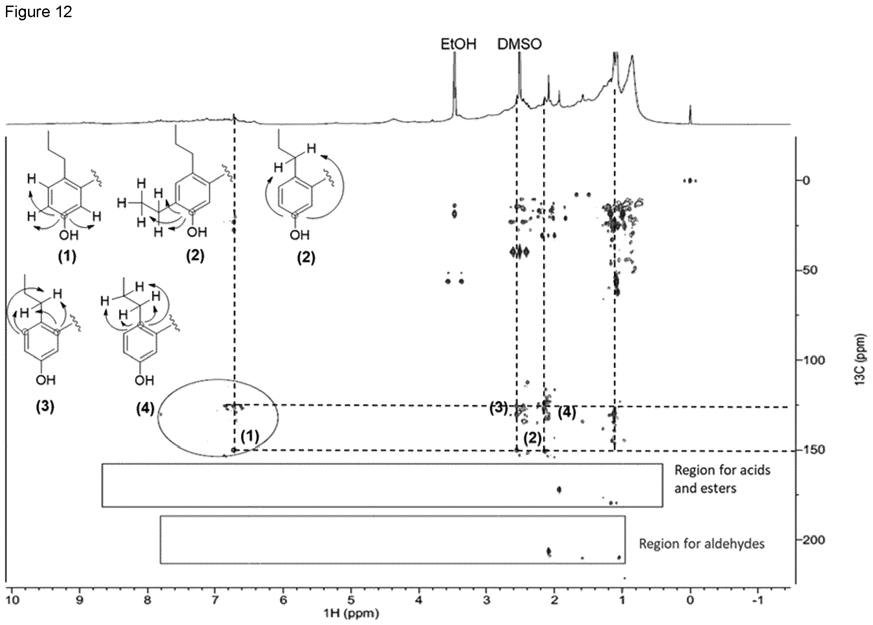

2D $^1$H-$^{13}$C HMBC NMR of 'Lignin oligomer' (Figure 12) is less busy in comparison to starting lignin, which indicates a more uniform structure. The presence of phenolic OH could be confirmed by cross peak at δC/δH 150/6.8 ppm (1), which displays interaction of aromatic protons to aromatic, quaternary, heteroatom bound carbon. The additional cross peaks at δC/δH 150/2.2 ppm and δC/δH 150/2.6 ppm are attributed to correlation of the same quaternary carbon atom with -CH2-groups of alkyl chains (2). These kinds of correlations haven't been seen in the starting kraft lignin, indicating methoxy group elimination and alkylation. Additional cross peaks at δC/δH 128/2.2 ppm (4) and δC/δH 128/2.6 ppm (3) show correlation of alkyl protons with aromatic carbons in position 3, 4, 5. The absence of a correlation between -CH3 group and aromatic carbons (δC/δH 125-150/0.8-0.9 ppm) shows elimination of methoxy groups .

In the HMBC, no additional signals of acids, esters or aldehydes relative to the starting material were observed.

NMR interpretation

The hydrogenated lignin oligomer sample has a lower total-OH content than the starting materials. Both aliphatic-OH and aromatic OH are reduced relative to the starting material. Ethanol can be distinguished as residual solvent left in the sample. The content of carboxylic-OH does not rise significantly. However, methoxy groups are drastically reduced beyond detection limit. The ratio of aromatic CH to aliphatic CH is decreased significantly from 2:1 to 1:7.

**Claims**

1.  Process for depolymerization of lignin comprising thermal conversion of an aqueous mixture having a pH of at least 9 comprising lignin, catalyst and primary alcohol in a non-oxidizing atmosphere at a temperature of at least 280°C.

2.  Process for depolymerization of lignin, comprising the steps of:

    a) Providing an aqueous mixture comprising:
    lignin, catalyst and primary alcohol having a pH of at least 9 in a non-oxidizing atmosphere,
    b) Thermal conversion of the lignin in the aqueous mixture at a temperature of at least 280°C, and
    c) Obtaining precipitated lignin oligomer.

3.  Process according to anyone of the preceding claims, wherein the thermal conversion in step b) is performed for 3 to 7 h after precipitation of lignin oligomer starts.

4.  Process of anyone of the preceding claims, wherein the aqueous mixture comprises

    5 to 25 wt.-% lignin,
    0.01 to 1.0 wt.-% catalyst,
    1 to 5 wt.-% base,
    5 to 45 wt.-% primary C1 to C4-alcohol,
    based on the total weight of the aqueous mixture.

5.  Process according to anyone of the preceding claims, wherein hydrothermal conversion is conducted at 80 to 150 bar at reaction temperature of 280°C to 400°C.

6.  Process according to anyone of the preceding claims, wherein the catalyst selected from the group consisting of Ru, Cu, Co, Ni, Pt, sulfides of said metals, oxides of said metals and mixtures thereof.

7.  Process according to anyone of the preceding claims, wherein the primary alcohol is selected from the group consisting of MeOH, EtOH, n-Propanol, n-Butanol and mixtures thereof.

8.  Process according to anyone of the preceding claims, wherein the non-oxidizing gas is N$_2$, argon, and/or H$_2$.

9.  Process according to anyone of the preceding claims, wherein the process provides lignin oligomers having a molecular weight of 250 to 750 g/mol, and/or having 2 to 10 aromatic moieties.

10. Process according to anyone of the preceding claims, wherein the process provides precipitated lignin oligomers having an oxygen content of less than 20 wt.-% based on the total weight of the precipitated lignin oligomers.

11. Process according to anyone of the preceding claims, wherein the catalyst, base, primary alcohol and/or unconverted lignin can be reintroduced in the process for depolymerization of lignin either in a batch or continuous process.

12. Process according to anyone of the preceding claims, wherein the depolymerized lignin-oligomer is further converted to polymers.

13. Process according to anyone of the preceding claims, wherein the depolymerized lignin-oligomer is further converted to polymers selected from the group consisting of polyesters, polyurethanes, polyamides and mixtures thereof.

14. Process according to anyone of the preceding claims, wherein the depolymerized lignin-oligomer is further converted to bio-based aromatics selected from the group consisting of toluene, benzene, xylenes and mixtures thereof.

**Patentansprüche**

1. Verfahren zur Depolymerisation von Lignin, umfassend thermische Umwandlung eines wässrigen Gemischs mit einem pH-Wert von wenigstens 9, das Lignin, Katalysator und primären Alkohol umfasst, in einer nichtoxidierenden Atmosphäre bei einer Temperatur von wenigstens 280°C.

2. Verfahren zur Depolymerisation von Lignin, umfassend die Schritte:

   (A) Bereitstellen eines wässrigen Gemischs, umfassend Lignin, Katalysator und primären Alkohol, mit einem pH-Wert von wenigstens 9 in einer nichtoxidierenden Atmosphäre,
   b) thermische Umwandlung des Lignins in dem wässrigen Gemisch bei einer Temperatur von wenigstens 280°C, und
   c) Erhalten von präzipitiertem Lignin-Oligomer.

3. Verfahren gemäß einem der vorstehenden Ansprüche, wobei die thermische Umwandlung bei Schritt b) für 3 bis 7 h nach dem Beginn der Präzipitation von Lignin-Oligomer durchgeführt wird.

4. Verfahren gemäß einem der vorstehenden Ansprüche, wobei das wässrige Gemisch umfasst:

   5 bis 25 Gew.-% Lignin,
   0,01 bis 1,0 Gew.-% Katalysator,
   1 bis 5 Gew.-% Base,
   5 bis 45 Gew.-% primären C1- bis C4-Alkohol,
   bezogen auf das Gesamtgewicht des wässrigen Gemischs.

5. Verfahren gemäß einem der vorstehenden Ansprüche, wobei hydrothermische Umwandlung bei 80 bis 150 bar bei einer Reaktionstemperatur von 280 °C bis 400 °C durchgeführt wird.

6. Verfahren gemäß einem der vorstehenden Ansprüche, wobei der Katalysator ausgewählt ist aus der Gruppe bestehend aus Ru, Cu, Co, Ni, Pt, Sulfiden der Metalle, Oxiden der Metalle und Gemischen davon.

7. Verfahren gemäß einem der vorstehenden Ansprüche, wobei der primäre Alkohol ausgewählt ist aus der Gruppe bestehend aus MeOH, EtOH, n-Propanol, n-Butanol und Gemischen davon.

8. Verfahren gemäß einem der vorstehenden Ansprüche, wobei das nichtoxidierende Gas $N_2$, Argon und/oder $H_2$ ist.

9. Verfahren gemäß einem der vorstehenden Ansprüche, wobei das Verfahren Lignin-Oligomere mit einem Molekulargewicht von 250 bis 750 g/mol und/oder mit 2 bis 10 aromatischen Einheiten bereitstellt.

10. Verfahren gemäß einem der vorstehenden Ansprüche, wobei das Verfahren präzipitierte Lignin-Oligomere mit einem Sauerstoffgehalt von weniger als 20 Gew.-%, bezogen auf das Gesamtgewicht der präzipitierte Lignin-Oligomere, bereitstellt.

11. Verfahren gemäß einem der vorstehenden Ansprüche, wobei der Katalysator, die Base, der primäre Alkohol und/oder nicht umgewandeltes Lignin in einem Batch- oder einem kontinuierlichen Verfahren in das Verfahren zur Polymerisation wiedereingeführt werden kann.

12. Verfahren gemäß einem der vorstehenden Ansprüche, wobei das depolymerisierte Lignin-Oligomer ferner zu Polymeren umgewandelt wird.

13. Verfahren gemäß einem der vorstehenden Ansprüche, wobei das depolymerisierte Lignin-Oligomer ferner zu Polymeren ausgewählt aus der Gruppe bestehend aus Polyestern, Polyurethanen, Polyamiden und Gemischen davon umgewandelt wird.

14. Verfahren gemäß einem der vorstehenden Ansprüche, wobei das depolymerisierte Lignin-Oligomer ferner zu Aromaten auf Biobasis ausgewählt aus der Gruppe bestehend aus Toluol, Benzol, Xylenen und Gemischen davon umgewandelt wird.

## Revendications

1. Procédé pour la dépolymérisation de lignine comprenant une conversion thermique d'un mélange aqueux ayant un pH d'au moins 9 comprenant de la lignine, un catalyseur et un alcool primaire dans une atmosphère non oxydante à une température d'au moins 280 °C.

2. Procédé pour la dépolymérisation de lignine, comprenant les étapes de :

   a) fourniture d'un mélange aqueux comprenant :
   de la lignine, un catalyseur et un alcool primaire ayant un pH d'au moins 9 dans une atmosphère non oxydante,
   b) conversion thermique de la lignine dans le mélange aqueux à une température d'au moins 280 °C, et
   c) obtention d'un oligomère de lignine précipité.

3. Procédé selon l'une quelconque des revendications précédentes, la conversion thermique dans l'étape b) étant réalisée pendant 3 à 7 h après le début de la précipitation d'oligomère de lignine.

4. Procédé selon l'une quelconque des revendications précédentes, le mélange aqueux comprenant

   5 à 25 % en poids de lignine,
   0,01 à 1,0 % en poids de catalyseur,
   1 à 5 % en poids de base,
   5 à 45 % en poids d'alcool primaire en C1 à C4,
   sur la base du poids total du mélange aqueux.

5. Procédé selon l'une quelconque des revendications précédentes, une conversion hydrothermale étant conduite à une pression de 80 à 150 bars à une température de réaction de 280 °C à 400 °C.

6. Procédé selon l'une quelconque des revendications précédentes, le catalyseur étant choisi dans le groupe constitué par Ru, Cu, Co, Ni, Pt, des sulfures desdits métaux, des oxydes desdits métaux et des mélanges correspondants.

7. Procédé selon l'une quelconque des revendications précédentes, l'alcool primaire étant choisi dans le groupe constitué par MeOH, EtOH, n-Propanol, n-Butanol et des mélanges correspondants.

8. Procédé selon l'une quelconque des revendications précédentes, le gaz non oxydant étant $N_2$, l'argon, et/ou Hz.

9. Procédé selon l'une quelconque des revendications précédentes, le procédé fournissant des oligomères de lignine ayant un poids moléculaire de 250 à 750 g/mole, et/ou ayant 2 à 10 groupements aromatiques.

10. Procédé selon l'une quelconque des revendications précédentes, le procédé fournissant des oligomères de lignine précipités ayant une teneur en oxygène inférieure à 20 % en poids sur la base du poids total des oligomères de lignine précipités.

11. Procédé selon l'une quelconque des revendications précédentes, le catalyseur, la base, l'alcool primaire et/ou la lignine non convertie pouvant être réintroduits dans le procédé pour dépolymérisation de lignine soit dans un procédé par lots, soit continu.

12. Procédé selon l'une quelconque des revendications précédentes, l'oligomère de lignine dépolymérisé étant en outre converti en polymères.

13. Procédé selon l'une quelconque des revendications précédentes, l'oligomère de lignine dépolymérisé étant en outre converti en polymères choisis dans le groupe constitué par des polyesters, des polyuréthanes, des polyamides et des mélanges correspondants.

14. Procédé selon l'une quelconque des revendications précédentes, l'oligomère de lignine dépolymérisé étant en outre converti en composés aromatiques biosourcés choisis dans le groupe constitué par le toluène, le benzène, les xylènes et des mélanges correspondants.

Fig 1

EP 4 069 666 B1

Figure 2

EP 4 069 666 B1

Figure 3

Figure 4

| Parameters | Lignin | Lignin oligomer |
|---|---|---|
| Mn, g/mol | 1400 | 350-650 |
| PDI | 7.3 | 1.5-2.3 |
| O/C | 0.46 | 0.15-0.28 |
| $T_g$, °C | 144.3 | 21.5 |
| Ether linkages | **Yes** | **No** or significantly decreased |
| Presence of aromatic ring | Yes | Yes |
| Presence of phenolic hydroxyl | Yes | Yes |
| Presence of methoxy groups | **Yes** | **No** or residual |
| Presence of carbonyl/ carboxylic groups | Yes | Yes |
| Presence of alkyl substituents in aromatic ring | Yes | Yes (**increased**) |
| Main type of monomer linkage | **C-O linkages** *[ether bond]* | **C-C linkage** *[methylene bridges (-CH(CH$_3$)-) or direct C-C linkage of aromatic cores]* |

Figure 5

| Parameters | Kraft lignin | Lignin oligomer | EP2975015 | WO2014/201325 | Konnerth et al. | Cheng et al. |
|---|---|---|---|---|---|---|
| Mn, g/mol | 1400 | 350-650 | n/a | 300-700 | n/a | 150-300 |
| PDI | 7.3 | 1.5-2.3 | n/a | < 2 | n/a | n/a |
| O/C | 0.46 | 0.15-0.28 | n/a | n/a | n/a | n/a |
| $T_g$, °C | 144.3 | 21.5 | liquids | liquids | liquids | liquids |
| Ether linkages | **Yes** | **No** or significantly decreased | n/a | n/a | n/a | n/a |
| Presence of aromatic ring | Yes | Yes | Yes | Yes | Yes | Yes |
| Presence of phenolic -OH | Yes | Yes | Yes | Yes | Yes | Yes |
| Presence of methoxy groups | **Yes** | **No** or residual | Yes | Yes | Yes | Yes |
| Presence of CO/COO -groups | Yes | Yes | yes | n/a | n/a | n/a |
| Presence of alkyl substituents in aromatic ring | Yes | Yes (**increased**) | Yes | Yes | Yes | n/a |
| Main type of monomer linkage | **C-O linkages** [ether bond] | **C-C linkage** [methylene bridges (-CH(CH₃)-) or direct C-C linkage of aromatic cores] | Only Monomers, no Oligomers | n/a | Only Monomers, no Oligomers | n/a |

EP 4 069 666 B1

Figure 6

EP 4 069 666 B1

ratio of aromatics to aliphatics (1:7)

DMSO

x Ethanol

EP 4 069 666 B1

Figure 7

ratio of aromatics to aliphatics (2:1)

Figure 8

Figure 9

Figure 10

EtOH    DMSO    EtOH

13C NMR chemical shift for possible lignin depolymerization products

Signale aus Lignoboost nicht zu erkennen.

1H (ppm)

13C (ppm)

EP 4 069 666 B1

Figure 11

Figure 12

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017048163 A
- WO 2017078582 A
- WO 2014168473 A
- WO 2012005677 A
- EP 2975015 A
- WO 2014201325 A
- US 4507172 A

- CA 2256923
- EP 3156409 A
- WO 2013070130 A
- DE 3901662
- WO 2012027767 A
- WO 2006038863 A
- US 20170355723 A

**Non-patent literature cited in the description**

- **KONNERTH et al.** Base promoted hydrogenolysis of lignin model compounds and organosolv lignin over metal catalysts in water. *Chemical Engineering Science,* 2015, vol. 123, 155-163
- **CHENG et al.** Producing jet fuel from biomass lignin: Potential pathways to alkylbenzenes and cycloalkanes. *Renewable and Sustainable Energy Reviews,* 2017, vol. 72, 673-722

- Green Liquor, Chemical Recovery. **BAJPAI, PRATIMA.** Biermann's Handbook of Pulp and Paper - Raw Material and Pulp Making. 2018, vol. 1, 2, 332
- Granata and Argyropoulos. *J. Agric. Food Chem.,* 1995, vol. 43 (6